# EUROPEAN PATENT APPLICATION

(11) **EP 4 675 262 A1**
(43) Date of publication of application: **07.01.2026**
(21) Application number: 24763725.9
(22) Date of filing: 20.02.2024
(51) Int. Cl.: G01N 21/84, A01K 43/00, G01N 21/95, G01N 33/08

(54) **EGG INSPECTION DEVICE**

(30) Priority: 01.03.2023 JP 2023031177
(71) Applicant: Nabel Co., Ltd., Kyoto-shi, Kyoto 601-8444 (JP)
(72) Inventor: YOKOSE, Kuniyuki, Kyoto-shi, Kyoto 601-8444 (JP)
(74) Representative: Müller Hoffmann & Partner
(86) International application number: PCT/JP2024/006107
(87) International publication number: WO 2024/181244

(57) **Abstract**

The present invention makes it possible to adjust the attitude of an egg and perform an accurate inspection of that egg, and is provided with an attitude adjustment unit 11 that adjusts an attitude of an egg E by lifting the egg E from an egg tray 10 using a plurality of elastic bodies 11a that surround a periphery of the egg E, a cap unit 12 that is placed in contact with an upper end portion of the egg E that has been lifted up by the attitude adjustment unit 11, a lifting unit 4 that lifts up the egg E using the elastic bodies 11a while the cap unit 12 is in contact with the egg E, a light irradiation unit 5 that irradiates light onto the egg E that has been lifted by the lifting unit 4, and an imaging unit 6 that captures a photographic image of the egg E lifted by the lifting unit 4.

## Description

### [Technical Field]

The present invention relates to an egg inspection apparatus.

### [Background Art]

Conventionally, an inspection is made of hatching eggs that are used in vaccine production and the like to verify whether each hatching egg is a good egg (i.e., a normal egg) or an abnormal egg. An inspection apparatus such as that shown, for example, in Patent Document 1 may be adopted in order to perform such inspections.

This inspection apparatus has a structure in which an inspection wheel on which an egg has been placed is raised using a wheel supporting stand so that the egg is positioned in an image acquisition tube. Light is then irradiated onto the egg inside this image acquisition tube and a photographic image of the egg irradiated with the light is captured.

However, in an inspection apparatus such as this, no consideration whatsoever is given to the attitude of the egg on the inspection wheel such as whether or not the egg is tilted. Because of this, if the egg is tilted when the inspection wheel is raised up and is placed inside the image acquisition tube, then it is not possible for an inspection of the size, location, and configuration and the like of an air cell to be performed accurately. Note that adjusting any tilting of an egg at the time when the egg is placed on the inspection wheel may be considered, however, there is a possibility that the egg will become tilted when the inspection wheel is being conveyed, or that the egg will become tilted when the inspection wheel is being raised.

### [Citation List]

### [Patent Literature]

[PIT 1] Japanese Patent No. 3998184

### [Summary of Invention]

### [Technical Problem]

The present invention was therefore conceived in order to solve the above-described problems, and it is an object thereof to enable the attitude of an egg to be adjusted so that an inspection of the egg can be accurately performed.

### [Solution to Problem]

In other words, an egg inspection apparatus according to the present invention is an egg inspection apparatus that inspects an egg held on an egg tray, and that is characterized in being provided with an attitude adjustment unit that adjusts an attitude of the egg by lifting the egg off the egg tray using a plurality of elastic bodies that surround a periphery of the egg, a cap unit that is placed in contact with an upper end portion of the egg that has been lifted up by the attitude adjustment unit, a lifting unit that lifts up the egg using the elastic bodies while the cap unit is in contact with the egg, a light irradiation unit that irradiates light onto the egg that has been lifted by the lifting unit, and an imaging unit that captures a photographic image of the egg that has been lifted by the lifting unit.

If this type of egg inspection apparatus is adopted, then because a cap unit is placed in contact with an upper end portion of each egg whose attitude has been adjusted by the attitude adjustment unit and, in this state, each egg is lifted up by a lifting unit, it is possible for each egg whose attitude has been adjusted to be lifted up while the attitude thereof is maintained. As a result, it is possible to inspect eggs whose attitude has been adjusted, and it is possible to make an accurate inspection of an egg such as, for example, the size, position, or configuration or the like of an air cell, i.e., whether the air cell is too large or whether the air cell positioning is unsuitable (i.e., is a sloping air cell or sidewards air cell). Moreover, because each lifting unit lifts an egg above the elastic bodies of the attitude adjustment unit while the egg is in contact with the cap unit, the elastic bodies do not get in the way when a photographic image of the egg is being captured by the imaging unit.

A specific aspect of the cap unit that may be considered is a structure in which the cap unit is formed so as to be able to move up or down while still in contact with the upper end portion of the egg.

A specific aspect of the lifting unit that may be considered is a structure in which the lifting unit is provided with a stage unit that is in contact with a lower end portion of the egg, and with an actuator that causes the stage unit to move up or down. In this structure it is desirable that the attitude adjustment unit be provided so as to be able to move relatively to the stage unit, and is moved up or down by the actuator, and that, when the attitude adjustment unit is moved up by the actuator, the attitude adjustment unit adjust the attitude of the egg by lifting up the egg and that, thereafter, the upward movement of the attitude adjustment unit be halted and the stage unit be then moved up by the actuator so that the egg is lifted up above the elastic bodies.

If this type of structure is adopted, then the actuator can be used in common by both the lifting units and the attitude adjustment units, and it is possible to solve the problem of the egg inspection apparatus being made more complex and larger in size as a result of adding the structure of the attitude adjustment units thereto.

### [Advantageous Effects of the Invention]

According to the present invention which is formed in the manner described above, it is possible to adjust the attitude of an egg so as to enable an inspection of the egg to be performed accurately.

### [Brief Description of Drawings]

[FIG. 1] FIG. 1 shows captured photographic images of good eggs (i.e., normal eggs) and abnormal eggs.
[FIG. 2] FIG. 2 is a plan view schematically showing a structure of an egg inspection apparatus according to an embodiment of the present invention.
[FIG. 3] FIG. 3 is a view seen from a conveying direction schematically showing the structure of the egg inspection apparatus of the same embodiment.
[FIG. 4] FIG. 4 is a view seen from an orthogonal direction relative to the conveying direction schematically showing the structure of the egg inspection apparatus of the same embodiment.
[FIG. 5] FIG. 5 is a functional structure diagram of a control mechanism of the egg inspection apparatus of the same embodiment.
[FIG. 6] FIG. 6 is a plan view showing a positional relationship between an egg holding unit, a stage unit, and a plurality of elastic bodies in the same embodiment.
[FIG. 7] FIG. 7 contains schematic views showing an attitude adjustment operation of the same embodiment.
[FIG. 8] FIG. 8 contains schematic views showing light emission patterns of the egg inspection apparatus of the same embodiment.
[FIG. 9] FIG. 9 shows (a) captured photographic images of a light emission pattern 1, and (b) captured photographic images of a light emission pattern 2.
[FIG. 10] FIG. 10 is a flowchart showing an inspection procedure of the egg inspection apparatus of the same embodiment.
[FIG. 11] FIG. 11 is a schematic diagram showing a structure of a cap unit of a variant embodiment.

### [Description of Embodiments]

Hereinafter, an embodiment of an egg inspection apparatus according to the present invention will be described with reference to the drawings. Note that, in order to simplify an understanding thereof, each of the drawings depicted below is shown schematically with omissions or enhancements made where these have been deemed appropriate. In addition, component elements that are the same in the respective drawings are indicated by the same descriptive symbols and any duplicated description thereof is omitted.

### [1. Structure of an Egg Inspection Apparatus 100]

An egg inspection apparatus 100 of the present embodiment irradiates light onto an egg (i.e., a hatching egg) E that is used, for example, in vaccine production so as to capture a photographic image thereof, and then, based on this captured photographic image, performs an inspection as to whether the egg is a good egg (i.e., a normal egg) that is suitable for vaccine production, or is an abnormal egg that is not suitable for vaccine production.

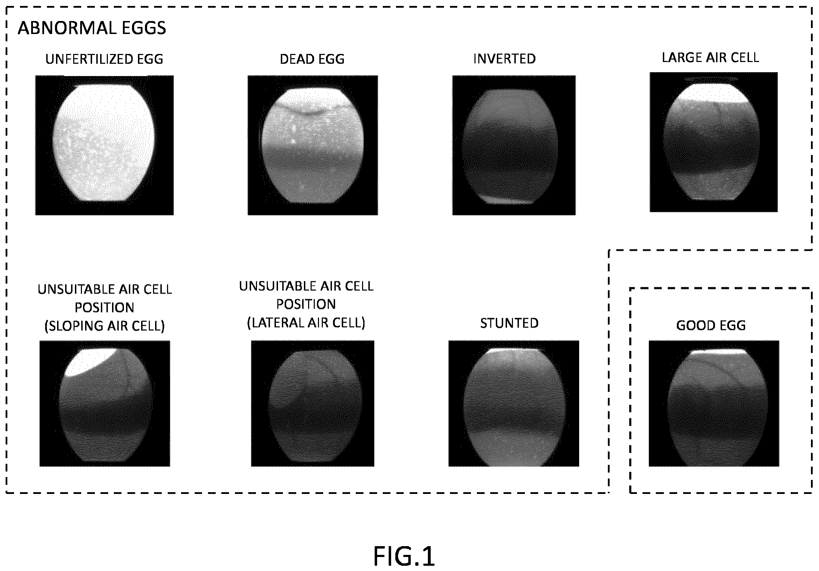

Here, as is shown in captured photographic images in FIG. 1, the categories for determining that an egg is abnormal include yolkless eggs (i.e., unfertilized eggs), dead eggs (eggs in which growth stopped mid-process, embryonic death eggs, or aborted eggs), upside-down (i.e., inverted) eggs, air cell is too large, air cell position is unsuitable (i.e., a sloping air cell or lateral air cell) and underdevelopment and the like.

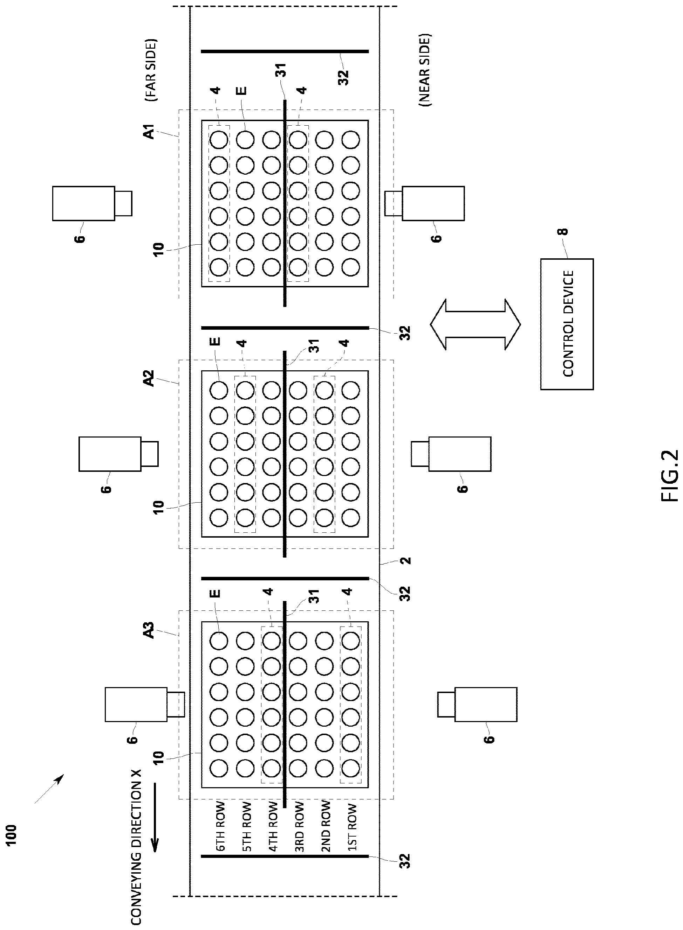

More specifically, as is shown in FIG. 2, the egg inspection apparatus 100 conveys an egg tray 10 that is holding a plurality of eggs E arranged in a plurality of rows along a conveyance path 2, and inspects the plurality of eggs E that are being held on the egg tray 10. In the following description, an egg tray 10 having six rows that each holds six eggs E is used as an example.

As is shown in FIG. 2, in the egg inspection apparatus 100 of the present embodiment, a plurality of inspection areas A1 to A3 are set in a conveying direction X on the conveyance path 2. In FIG. 2, an example is shown in which three inspection areas A1 to A3, namely, a first inspection area A1, a second inspection area A2, and a third inspection area A3 are set. Moreover, each of the inspection areas A1 to A3 is divided into a near side and a far side by a light shielding plate 31.

Here, in the respective inspection areas A1 to A3, the eggs E in the egg trays 10 being conveyed are separated by the light shielding plates 31 into a first row through a third row of eggs E on the near side, and a fourth row through a sixth row of eggs E on the far side. In other words, the eggs E in the first through third rows of each egg tray 10 are conveyed on the near side of the light shielding plate 31 in the respective inspection areas A1 to A3, while the eggs E in the fourth through sixth rows of each egg tray 10 are conveyed on the far side of the light shielding plate 31 in the respective inspection areas A1 to A3. Note that a lower end of each light shielding plate 31 is set at a height at which it is unable to come into contact with the eggs E being conveyed in the egg trays 10.

In addition, light shielding plates 32 are provided on both sides in the conveying direction X of the respective inspection areas A1 to A3. Note that the respective inspection areas A1 to A3 are covered by a common housing (not shown in the drawings), and these light shielding plates 32 are provided for the purposes of reducing ambient light entering via the entrance and exit of the conveyance path 2 in the housing, and of reducing the effects of light on any of the respective inspection areas A1 to A3 from any other of the inspection areas A1 to A3. Note also that a lower end of each light shielding plate 32 is set at a height at which it is unable to come into contact with the eggs E being conveyed in the egg trays 10.

In addition, as is shown in FIG. 2 through FIG. 4, for each of the inspection areas A1 to A3, the egg inspection apparatus 100 is also provided with lifting units 4 that lift up a plurality of eggs E that are disposed in predetermined rows, light irradiation units 5 that irradiate light onto the plurality of eggs E that have been lifted up by the lifting units 4, and imaging units 6 that capture photographic images of the plurality of eggs E that have been lifted up by the lifting units 4.

The lifting units 4 of the present embodiment are provided respectively on the near side and far side of the light shielding plates 31 in each of the inspection areas A1 to A3. Moreover, the lifting units 4 are formed such that the plurality of eggs E that they respectively lift up to a predetermined inspection position R are located in mutually different rows in the plurality of inspection areas A1 to A3. More specifically, the lifting units 4 of the first inspection area A1 are formed so that they lift up the plurality of eggs E that are located in the third row and the sixth row of the egg tray 10. The lifting units 4 of the second inspection area A2 are formed so that they lift up the plurality of eggs E that are located in the second row and the fifth row of the egg tray 10. The lifting units 4 of the third inspection area A3 are formed so that they lift up the plurality of eggs E that are located in the first row and the fourth row of the egg tray 10.

Furthermore, the lifting units 4 are disposed below the conveyance path 2, and are provided so as to be able to move up or down relative to an egg tray 10 on the conveyance path 2. In a state in which an egg tray 10 is stationary in each one of the respective inspection areas A1 to A3 on the conveyance path 2, each lifting unit 4 moves upwards from below the egg tray 10 so as to be in contact with a lower portion of the plurality of eggs E that are located in a predetermined row of the egg tray 10, and then lifts up this plurality of eggs E. More specifically, each lifting unit 4 is provided with stage units 41 that are provided respectively for the plurality of eggs E, a connecting component 42 on which the stage units 41 are disposed, and an actuator 43 such as, for example, an air cylinder or the like that causes the connecting component 42 to move up or down. The actuator 43 is controlled by a control unit 81 of a control device 8 (described below) and causes the plurality of stage units 41 to move up or down relative to the egg trays 10.

The light irradiation units 5 include a plurality of light sources that are provided so as to correspond to the plurality of eggs E which are arranged in a row, and irradiates light into the interior of the plurality of eggs E that are located in the predetermined inspection position R. In the present embodiment, a structure is adopted in which light is irradiated from above and below onto each of the plurality of eggs E that are arranged in a row. In other words, a structure is adopted in which a light source 51 and a light source 52 are provided respectively above and below each egg E.

More specifically, the light irradiation units 5 include a plurality of upper light sources 51 that irradiate light from above onto the plurality of eggs E that have been lifted up to the inspection position R by the lifting units 4, and a plurality of lower light sources 52 that irradiate light from below onto the plurality of eggs E that have been lifted up to the inspection position R by the lifting units 4. The upper light sources 51 and lower light sources 52 are provided so as to correspond to the lifting units 4 in each of the inspection areas A1 to A3. Note that the plurality of upper light sources 51 and the plurality of lower light sources 52 are controlled by the control unit 81 of the control device 8 (described below), and light emission patterns thereof (i.e., when each light source is turned on or off) are also controlled so as to be in mutual synchronization with each other.

The plurality of upper light sources 51 are provided above the plurality of eggs E that are lifted up by the lifting units 4. Here, a cap-shaped light shielding unit 7 that is placed in contact with the upper end portions of the eggs E that have been lifted up by the lifting units 4 so as to enclose and light-shield these upper end portions is provided in each upper light source 51. The light shielding units 7 are formed from an elastically deformable material so as to absorb any irregularities in the shapes of the eggs E. In addition, each upper light source 51 irradiates light from above onto the egg interior through a space that is formed inside the light shielding unit 7. Furthermore, each upper light source 51 is provided so as to be able to move up or down by means of ascent/descent guide units 9.

The plurality of lower light sources 52 are provided respectively inside the plurality of stage units 41 in each lifting unit 4. Here, each stage unit 41 includes an annular contact unit 411 that is placed in contact with the lower end portion of an egg E. In addition, each lower light source 52 irradiates light from below onto the egg interior through a space that is formed inside the contact unit 411 of the stage unit 41.

In the present embodiment, a structure is adopted in which the lifting units 4 lift the plurality of eggs E up to the predetermined inspection position R and, in a state in which the plurality of eggs E are sandwiched between the stage units 41 and the light shielding units 7, the eggs E are able to be rotated by predetermined degrees. Here, a structure is adopted in which the eggs E are able to be rotated in steps of 90 degrees (i.e., 0 degrees → 90 degrees → 180 degrees → 270 degrees).

More specifically, each stage unit 41 is provided so as to be able to rotate relative to the connecting component 42. These stage units 41 are mutually connected together by means of rotation transmission units 44 that adopt toothed belts or the like. As a result of the rotation transmission units 44 being driven by an actuator 45 such as a motor or the like, the plurality of stage units 45 arranged in a row are rotated in mutual synchronization in 90-degree steps. Note that the actuator 45 is controlled by the control unit 81 of the control device 8 (described below) so as to cause the plurality of stage units 41 to rotate.

The imaging units 6 capture photographic images of the plurality of eggs E that have been lifted up to the inspection position R by the lifting units 4. The imaging units 6 of the present embodiment are formed by color cameras having imaging elements such as, for example, CCD or CMOS.

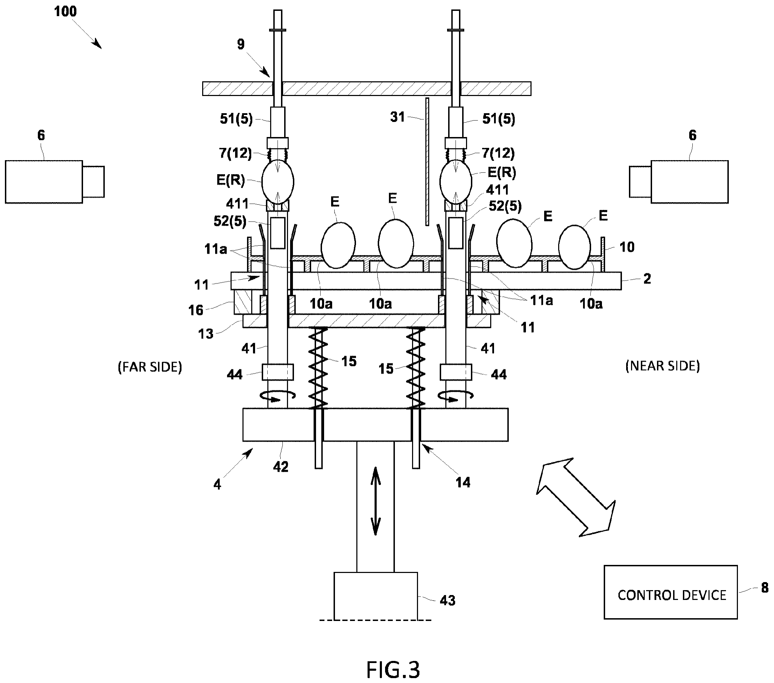

More specifically, as is shown in FIG. 2 and FIG. 3, an imaging unit 6 is provided respectively on both the near side and the far side of each light shielding plate 31 in the respective inspection areas A1 to A3. Each imaging unit 6 is positioned so as to be able to capture photographic images of the plurality of eggs E with the light shielding plates 31 used as a background. For example, on the near side of the first inspection area A1, an imaging unit 6 captures photographic images of a plurality of eggs E in the third row taking the light shielding plate 31 as a background, while on the far side of the first inspection area A1, an imaging unit 6 captures photographic images of a plurality of eggs E in the sixth row taking the light shielding plate 31 as a background. In addition to these, the relative positions between each imaging unit 6 and the plurality of eggs E in the rows whose photographic images are captured by these respective imaging units 6 are set so as to be mutually the same as each other (see FIG. 2). Note that the imaging units 6 perform the operations to capture photographic images of the plurality of eggs E under the control of the control unit 81 of the control device 8 (described below).

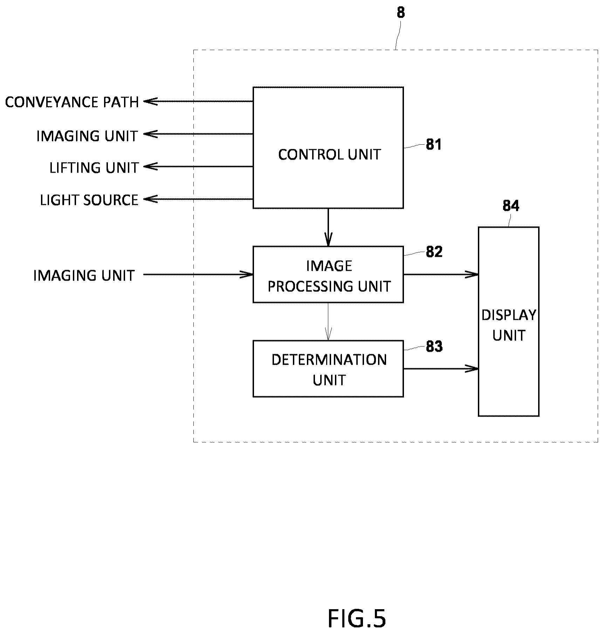

Moreover, as is shown in FIG. 5, the photographic images captured by the imaging units 6 are transmitted to an image processing unit 82 of the control device 8. After the photographic images have undergone image processing in the image processing unit 82, a determination is made by a determination unit 83 as to whether or not each egg is a normal egg or an abnormal egg. In a case in which an egg is determined to be an abnormal egg, it is also possible for a further determination to be made as to the type of abnormality of the abnormal egg. Furthermore, it is also possible to adopt a structure in which photographic images captured by the imaging units 6 are displayed on a display unit 84 such as a display monitor or the like so as to enable a user to determine from those photographic images whether an egg is a good egg or an abnormal egg.

### [2. Function for Adjusting an Attitude of an Egg E]

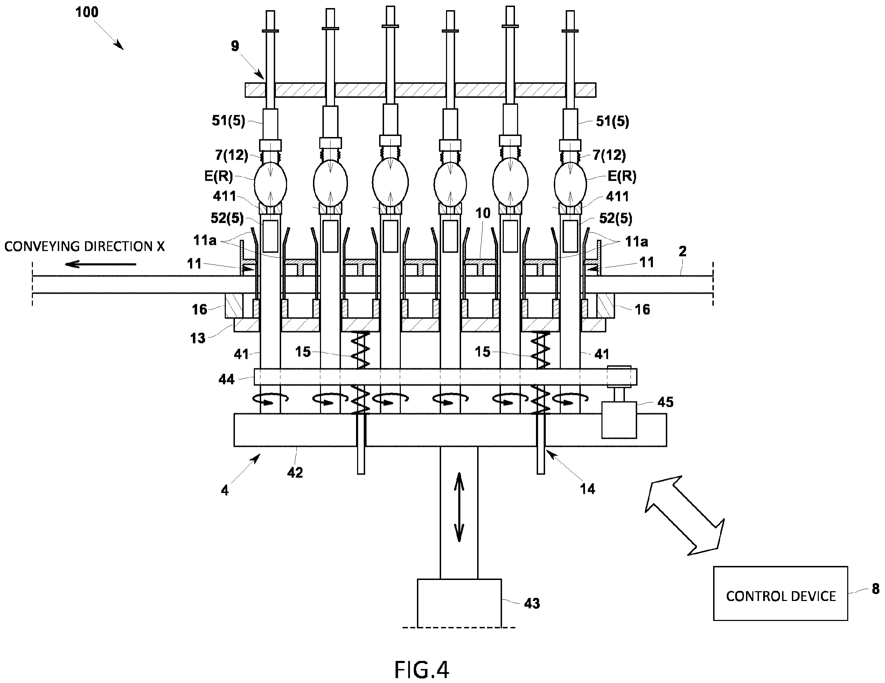

Moreover, as is shown in FIG. 3 and FIG. 4, the egg inspection apparatus 100 of the present embodiment is additionally provided with a plurality of attitude adjustment units 11 that lift up the eggs E from the egg tray 10 and then adjust the attitude of the eggs E, and with a plurality of cap units 12 that are placed in contact with the upper end portion of the eggs E that have been lifted up by the plurality of attitude adjustment units 11.

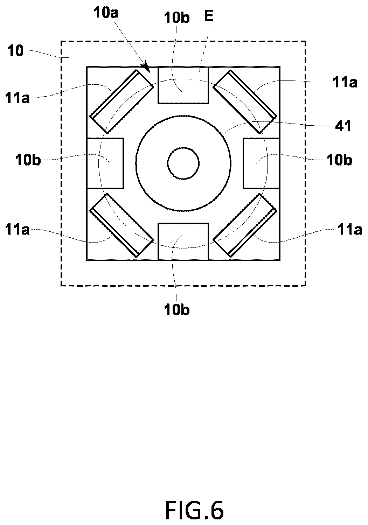

The respective attitude adjustment units 11 are provided so as to correspond individually to the respective stage units 41, and are formed so as to include a plurality of elastic bodies 11a that surround a periphery of the eggs E. More specifically, as is shown in FIG. 6, the plurality of elastic bodies 11a of each attitude adjustment unit 11 may be provided, for example, equidistantly from each other around the periphery of an egg E, and may be formed, for example, by a leaf spring or by another type of spring component. Moreover, the plurality of elastic bodies 11a of each attitude adjustment unit 11 are provided so as to surround each stage 41 when looked at in plan view.

In the present embodiment, egg holding units 10a that hold the individual eggs E on the egg tray 10 have a rectangular shape when looked at in plan view, and egg seat portions 10b are provided in a central portion of each side of each egg holding portion 10a. Four elastic bodies 11a are provided in the gaps formed between the four corner portions (i.e., in the gaps on either side of each egg seat portion 10b) of each egg holding portion 10a. Note that the elastic bodies 11a of the present embodiment all have the same configuration, however, they may also be formed so as to have mutually different configurations.

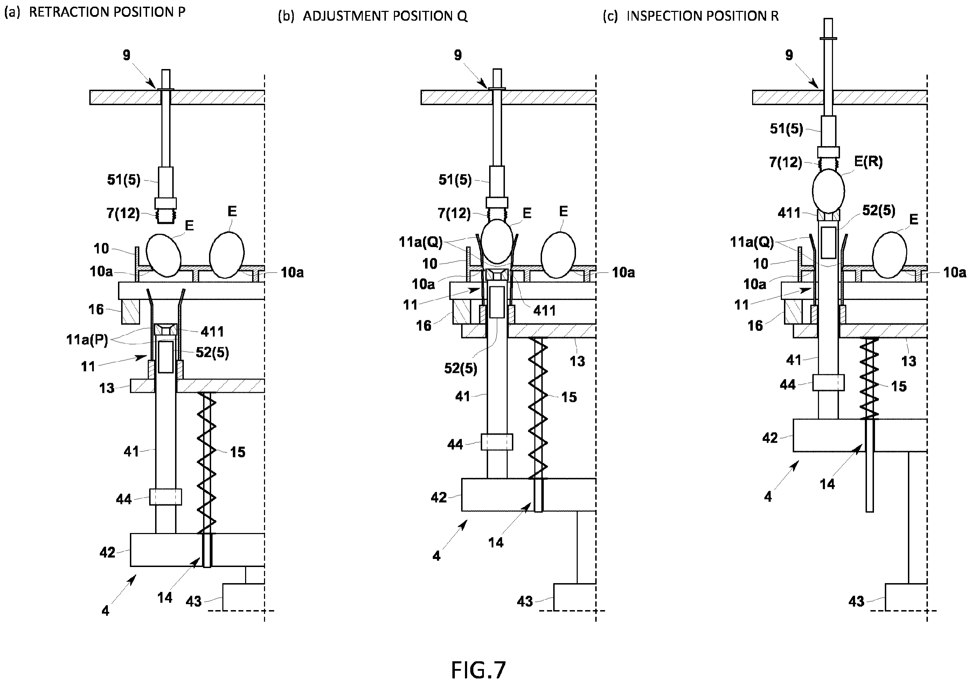

The plurality of elastic bodies 11a are formed so as to be able to move up or down relatively to the eggs E held on the egg tray 10. More specifically, the plurality of elastic bodies 11 move up or down between a retraction position P (see FIG. 7 (a)) where they are retracted below the egg tray 10 so as not to interfere with the conveying of the egg tray 10, and an adjustment position Q (see FIG. 7 (b)) where they protrude above the egg tray 10 and lift an egg E up from the egg tray 10 so as to adjust the attitude of that egg E. Note that, at the adjustment position Q, the respective elastic bodies 11a are in a state of protruding above the egg tray 10 through the gaps in the corner portions of the egg holding portion 10a.

As the plurality of elastic bodies 11a move upwards to the adjustment position Q, they come into contact with an egg E, and become elastically deformed at the same time as they lift the egg E up from the egg holding portion 10a. As a result, the attitude of the egg E is adjusted by straightening the egg E so that any tilting thereof is reduced.

Moreover, as is shown in FIG. 3 and FIG. 4, a structure is adopted in which each attitude adjustment unit 11 is provided so as to be able to move relatively to the respective stage units 41, and is moved upwards or downwards by the actuator 43 of the lifting unit 4.

More specifically, the plurality of attitude adjustment units 11 are supported by a supporting component 13 that is located below the conveyance path 2, and this supporting component 13 is provided so as to be able to move in an up-down direction relatively to the connecting component 42. Movement of the supporting component 13 in the up-down direction relative to the connecting component 42 is guided by ascent/descent guide units 14, and elastic components 15 are provided between the supporting component 13 and the connecting component 42.

The supporting component 13 is raised together with the connecting component 42 by the actuator 43 until it comes up against stopper components 16 that are disposed at predetermined positions. The supporting components 13 stop their upward movement once they hit against the stopper components 16 irrespective of the upward movement of the connecting component 42. Note that when the supporting component 13 comes up against the stopper units 16, the plurality of elastic bodies 11a are located at the adjustment position Q.

A cap unit 12 is provided respectively for each stage unit 41, and these cap units 12 maintain the attitude of the eggs E once the attitude thereof has been adjusted by the respective attitude adjustment unit 11. Each cap unit 12 is placed in contact with the upper end portion of an egg E whose attitude has been adjusted after having been lifted up by the relevant attitude adjustment unit 11 so as to surround this upper end portion. More specifically, each cap unit is placed in contact with the upper end portion of an egg E that has been lifted up by the plurality of elastic bodies 11a which are at the adjustment position Q so as to surround this upper end portion.

The cap units 12 of the present embodiment are formed by the light shielding units 7 provided on the upper light sources 51. These cap units 12 are also formed so as to be able to move up or down while remaining in contact with the upper end portion of an egg E. More specifically, the cap units 12 are able to move up or down as a result of the upper light sources 51 being formed so as to be able to be raised or lowered via the ascent/descent guide units 9.

Because the cap units 12 are formed so as to be able to move up or down, the lifting units 4 are able to lift an egg E, while the cap unit 12 is in contact therewith, upwards beyond the elastic bodies 11a which are at the adjustment position Q. More specifically, the stage units 41 are able to lift up an egg E, while the cap unit 12 is in contact therewith, above the elastic bodies 11a, which are at the adjustment position Q, to the inspection position R. Here, the lower end portion of each egg E that is located at the inspection position R is positioned above an upper end of the elastic bodies 11a which are at the adjustment position Q.

### [3. Attitude Adjustment Operation]

Next, an operation performed by the attitude adjustment units 11 to adjust the attitude of the eggs E will be described.

When the connecting component 42 is raised by the actuator 43 from a state in which the plurality of elastic bodies 11a of the respective attitude adjustment units 11 are located at the retraction position P (see FIG. 7 (a)), the plurality of elastic bodies 11a of the respective attitude adjustment units 11 are raised together with the connecting component 42. The plurality of elastic bodies 11a then adjust the attitude of the eggs E in the egg holding portions 10a of the egg tray 10 by lifting up these eggs E. At this time, the plurality of elastic bodies 11a of the respective attitude adjustment units 11 adjust the attitude of the eggs E while being elastically deformed due to the weight of the eggs E.

When the plurality of elastic bodies 11a of the respective attitude adjustment units 11 have been raised to the adjustment position Q (see FIG. 7 (b)), each cap unit 12 is in contact with the upper end portion of an egg E whose attitude has been adjusted by the plurality of elastic bodies 11a. Here, each egg E is sandwiched between the plurality of elastic bodies 11a at the adjustment position Q and the cap unit 12, so that the attitude of the egg after this adjustment is maintained.

When the plurality of elastic bodies 11a of the respective attitude adjustment units 11 are raised to the adjustment position Q, the supporting component 13 that is supporting the respective attitude adjustment units 11 comes up against the stopper components 16, so that the plurality of elastic bodies 11a of the respective attitude adjustment units 11 are unable to rise beyond that point. Note that the stopper components 16 of the present embodiment are formed by components that make up the conveyance path 2, however, it is also possible to provide stopper components that are separate from the conveyance path 2.

Thereafter, when the connecting component 42 is raised by the actuator 43, in spite of the upward movement of the respective attitude adjustment units 11 having already stopped, the connecting component 42 continues to rise as the elastic components 15 become progressively more compressed. As a result, the stage units 41 provided on the connecting component 42 ascend inside the plurality of elastic bodies 11a at the adjustment position Q. Next, each stage unit 41 receives an egg whose attitude has been adjusted from the plurality of elastic bodies 11a (i.e., from the relevant attitude adjustment unit 11), and lifts that egg E above the plurality of elastic bodies 11a which are located at the adjustment position Q to the inspection position R (see FIG. 7 (c)). At this time, each cap unit 12 (i.e., shielding unit 7) that is in contact with the upper end portion of an egg E is raised upwards together with the egg E while being maintained in that state of contact by the ascent/descent guide units 9. In other words, each egg E is sandwiched between the stage unit 41 and the cap unit 12, and the attitude of that egg E after adjustment is maintained while the egg E is being lifted up.

In an operation to restore an egg E that has been inspected to the egg tray 10, the connecting component 42 is firstly lowered by the actuator 43. This causes the stage units 41 to also move downwards so that the eggs E sitting on the stage units 41 are transferred to the plurality of elastic bodies 11a (i.e., to the respective attitude adjustment units 11) which are at the adjustment position Q. Next, when the connecting component 42 is lowered by the actuator 43, as the stage units 41 are lowered the supporting component 13 separates from the stopper components 16, and the plurality of elastic bodies 11a (i.e., by the respective attitude adjustment units 11) start to move downwards together with the connecting component 42. As a result, the eggs E being supported by the plurality of elastic bodies 11a (i.e., the respective attitude adjustment units 11) are transferred to the egg holding portions 10a of the egg tray 10. Thereafter, the connecting component 42 is lowered further by the actuator 43 so that the plurality of elastic bodies 11a (i.e., the respective attitude adjustment units 11) are lowered to the retraction position P.

### [4. Structure of the Control device 8]

As is shown in FIG. 5, the control device 8 of the egg inspection apparatus 100 of the present embodiment controls operations such as operating and halting the conveyance path 2, up-down movements and rotational movements of the lifting units 4, turning the light irradiation units 5 on and off, and capturing photographic images using the imaging unit 6, and the like. Note that, as is described above, by controlling the up-down movements of the lifting units 4, the attitude adjustment performed by the attitude adjustment units 11 is also achieved.

Note that the control device 8 is formed by a computer that is equipped with a CPU, internal memory, input/output interfaces, and AD converters and the like. As a result of the CPU and peripheral devices operating in mutual collaboration with each other based on an inspection program that is stored in the internal memory, the control device 8 performs the functions of the control unit 81 that performs the overall control of the inspection apparatus 10, the image processing unit 82, the determination unit 83, and the display unit 84 and the like. These functions of the control device 8 may be performed by a single physical computer, or they may each be performed by a plurality of physically separate computers.

More specifically, the control unit 81 controls the light irradiation units 5, and switches these between a plurality of light emission patterns so that two light sources that are mutually adjacent to each other do not both emit light at the same time.

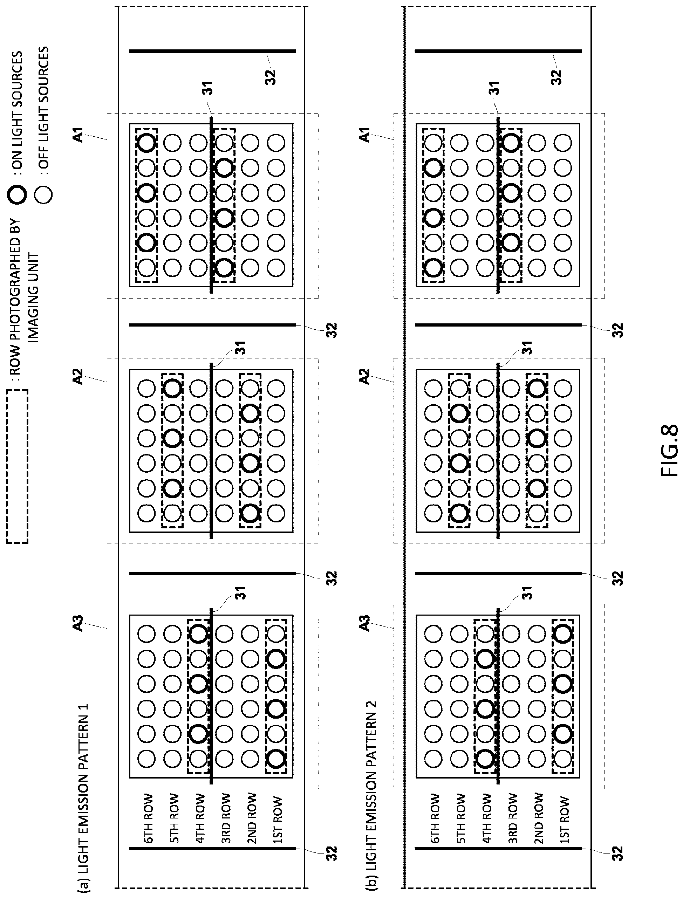

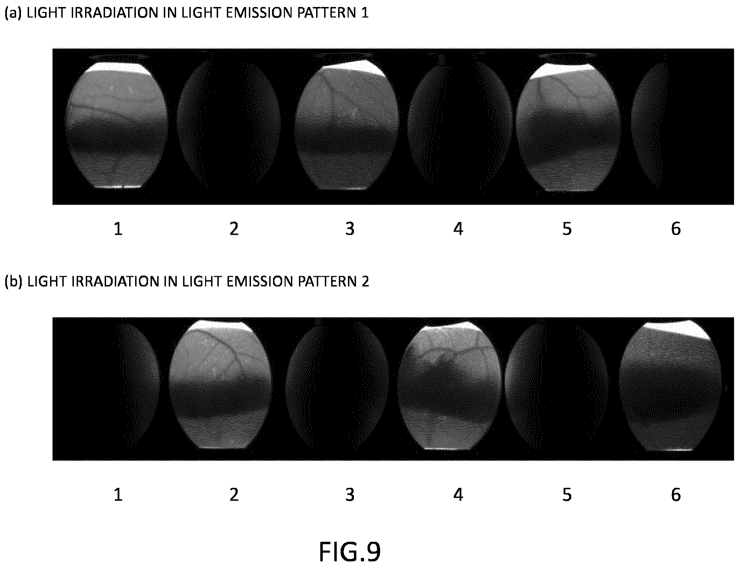

In the present embodiment, as is shown in FIG. 8, the light irradiation units 5 that are arranged in a row are switched between a light emission pattern (see FIG. 8 (a)) in which the light sources 51 and 52 in the odd-numbered rows as seen from the corresponding imaging units 6 are made to emit light (i.e., are turned on), and a light emission pattern (see FIG. 8 (b)) in which the light sources 51 and 52 in the even-numbered rows are made to emit light (i.e., are turned on). Moreover, as is shown in FIG. 9, the control unit 81 controls the imaging units 6 so that photographic images of a plurality of the eggs E are captured in each of a plurality of light emission patterns. In the present embodiment, by capturing photographic images in a light emission pattern 1 and photographic images in a light emission pattern 2, it is possible to capture photographic images of all of the plurality of eggs E that are arranged in predetermined rows.

### [5. Overall Operation of the Egg Inspection Apparatus 100]

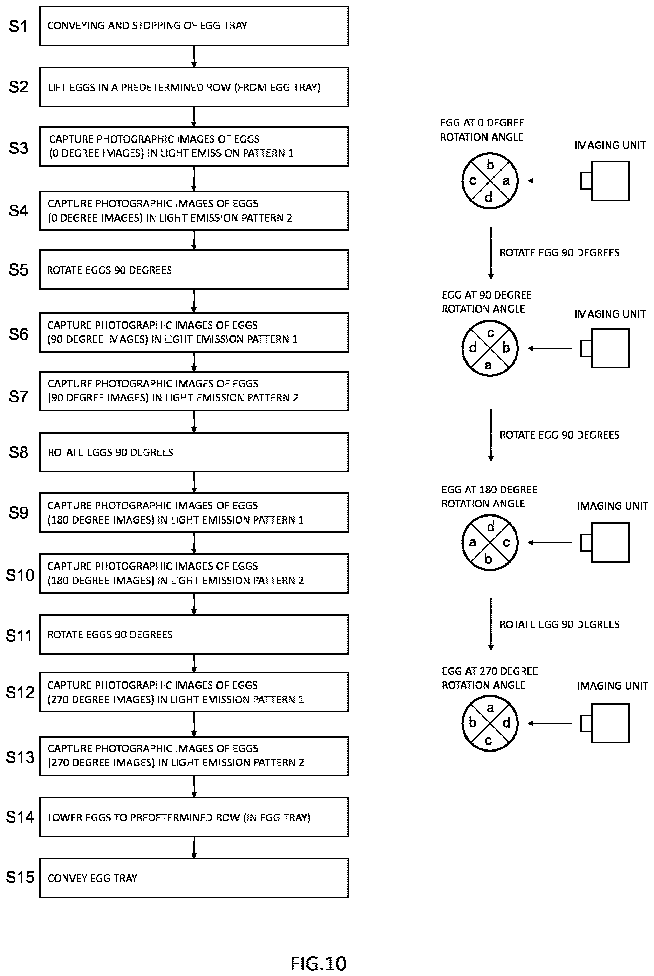

Next, an operation of the egg inspection apparatus 100 together with functions of the control device 8 will be described with reference to FIG. 10.

The control unit 81 controls the conveyance path 2 so that a plurality of egg trays 10 are conveyed to, and are stopped in, the respective inspection areas A1 to A3 (step S1). Next, the plurality of eggs E disposed in predetermined rows are lifted by the lifting units 4 provided in the respective inspection areas A1 to A3 (step S2: see FIG. 3).

Here, in the operation performed by the lifting units 4 to lift the plurality of eggs E, the attitudes of the plurality of eggs E are adjusted as a result of the eggs E being lifted up by the plurality of attitude adjustment units 11 (see FIG. 7 (b)), and this plurality of eggs E whose attitudes have been adjusted are then lifted up further by the lifting units 4 as far as the inspection position R (see FIG. 7 (c)). In this way, in the present embodiment, the adjustment of the attitude of the eggs E and the lifting of the eggs E to the inspection position R are performed continuously at one location.

Note that, in the first inspection area A1, the plurality of eggs E in the sixth row and the plurality of eggs E in the third row are lifted up, while in the second inspection area A2, the plurality of eggs E in the fifth row and the plurality of eggs E in the second row are lifted up, and in the third inspection area A3, the plurality of eggs E in the fourth row and the plurality of eggs E in the first row are lifted up.

Next, the control unit 81 turns on the odd-numbered light sources 51 and 52 from the left side as seen from the imaging units 6 (i.e., places these in the light emission pattern 1: see FIG. 8 (a)). In a state in which light is irradiated onto the odd-numbered eggs E from the left side (i.e., in a state in which the interior of each egg E has become luminous), the imaging units 6 capture photographic images of the plurality of eggs E (step S3: see FIG. 9 (a)). Thereafter, the control unit 81 turns on the even-numbered light sources 51 and 52 as seen from the imaging units 6 (i.e., places these in the light emission pattern 2: see FIG. 8 (b)). In a state in which light is irradiated onto the even-numbered eggs E from the left side (i.e., in a state in which the interior of each egg E has become luminous), the imaging units 6 capture photographic images of the plurality of eggs E (step S4: see FIG. 9 (b)). As a result, photographic images (i.e., 0-degree photographic images) of the respective light emission patterns in a 0-degree state (i.e., in the state in which the eggs E were conveyed in) are captured.

Next, the control unit 81 rotates the stage units 41 of the lifting units 4 a plurality of times by 90 degrees each time. After each rotation, the control unit 81 causes the light irradiation units 5 to emit light in the light emission pattern 1 and the light emission pattern 2, and photographic images of the plurality of eggs E are captured by the imaging units 6 in each light emission pattern (steps S5 to S13). As a result, photographic images (i.e., 90 degree photographic images of each light emission pattern in a state in which the stage units 41 have been rotated 90 degrees, photographic images (i.e., 180 degree photographic images of each light emission pattern in a state in which the stage units 41 have been rotated 180 degrees, and photographic images (i.e., 270 degree photographic images of each light emission pattern in a state in which the stage units 41 have been rotated 270 degrees are captured. In this way, in the present embodiment, the adjustment of the attitude of each egg E, and the lifting of the eggs E to the inspection position R and the rotation thereof are all performed in one location.

After photographic images in each light emission pattern at the above-described four different angles (i.e., in four directions) have been captured, the control unit 81 lowers the lifting units 4 so as to restore the plurality of eggs E that had been lifted up back to the egg trays 10 (step S14). Here, when the lifting units 4 are lowered, the eggs E are transferred from the stage units 41 to the plurality of elastic bodies 11a of the attitude adjustment units 11 and, thereafter, the eggs E are transferred from the plurality of elastic bodies 11a to the egg holding portions 10a of the egg trays 10.

In addition, the photographic images captured at each light emission pattern at the four different angles (i.e., in the four directions) are transmitted to the image processing unit 82. After image processing is performed thereon by the image processing unit 82, a determination is made by the determination unit 83 as to whether each egg is a normal egg or an abnormal egg. As a result, the inspection of the plurality of eggs E arranged in predetermined rows in the plurality of egg trays 10 is ended.

When the above-described operation is ended in the first inspection area A1, the control unit 81 controls the conveyance path 2 so that the egg tray 10 is conveyed to the next inspection area, which is the second inspection area A2, and is then stopped. When the above-described operation is ended in the second inspection area A2, the control unit 81 controls the conveyance path 2 so that the egg tray 10 is conveyed to the next inspection area, which is the third inspection area A3, and is then stopped. When the above-described operation is ended in the third inspection area A3, the control unit 81 controls the conveyance path 2 so that the egg tray 10 is conveyed to the downstream side of the third inspection area A3. Note that, in order to improve the processing performance of the egg inspection apparatus 100, it is also possible for the control unit 81 to control the conveyance path 2 so that the egg tray 10 is conveyed to the next inspection and then stopped at the same time as the image processing by the image processing unit 82 and the determination by the determination unit 83 are being performed.

By performing the above-described series of operations, the egg tray 10 can be moved to the plurality of inspection areas A1 to A3 that have been set on the conveyance path 2. As a result, the plurality of eggs E that are placed in mutually different rows can be inspected in sequence, so that all of the eggs E being held on the egg tray 10 can be inspected. Moreover, by feeding a plurality of egg trays 10 to the conveyance path 2, it is possible to inspect in sequence the plurality of eggs E that have been placed in each row of the plurality of egg trays 10.

### [6. Effects Obtained from the Present Embodiment]

According to the egg inspection apparatus 100 of the present embodiment, because the cap units 12 are placed in contact with an upper end portion of the eggs E whose attitudes have been adjusted by the attitude adjustment units 11 and, in this state, the eggs E are then lifted up by the lifting units 4, it is possible for the eggs E to be lifted up while the attitude-adjusted state of the eggs E is maintained. As a result, it is possible to inspect eggs E whose attitude has been adjusted, and it is possible to make an accurate inspection of an egg E, such as of the size, position, or configuration or the like of an air cell, such as, for example, whether the air cell is too large or whether the air cell positioning is unsuitable (e.g., is a sloping air cell or lateral air cell). Moreover, because the lifting units 4 lift the eggs E above the elastic bodies 11a which are located at the adjustment position Q while the eggs E are in contact with the cap units 12, the elastic bodies 11a do not get in the way when photographic images of the eggs E are being captured by the imaging units 6.

Moreover, in the present embodiment, a structure is adopted in which light is irradiated onto a plurality of eggs E that are arranged in a row and photographic images of this plurality of eggs E are then captured. Accordingly, it is possible to capture photographic images of the plurality of eggs E while using a simplified apparatus structure. In particular, in the present embodiment, it is possible not only to adjust the attitudes of the plurality of eggs E arranged in a row, but because photographic images of the plurality of eggs E are captured using light emission patterns from two mutually adjacent light sources 51 and 52 that do not both emit light simultaneously, compared with a case in which a plurality of light sources are all turned on, blood vessels that are present near side surfaces of the eggs are more easily visible due to light from the adjacent eggs E, and there is no change in the coloring thereof. Moreover, of the plurality of eggs E, between one row of eggs E and another row of eggs E onto which light is irradiated, there are rows of eggs E onto which light is not irradiated so that the eggs E in one row onto which light is irradiated are not affected by the light from the eggs E in the other row onto which light is irradiated. As a result, it is simple to ascertain features relating to the blood vessels or coloring of an egg which show the growth state of the eggs, and an accurate inspection of each individual egg E can be performed.

### [7. Variant Embodiments of the Present Invention]

Note that the present invention is not limited to the above-described embodiment.

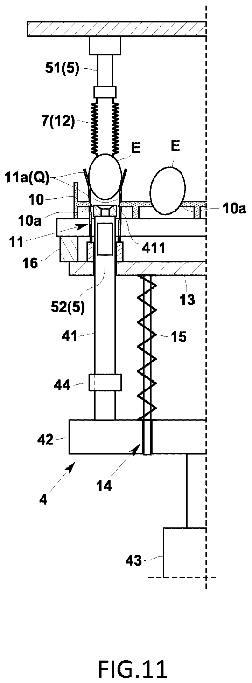

For example, the cap units 12 of the above-described embodiment are formed so as to be able to move up or down while in a state of contact with the upper end portion of the eggs E, however, it is also possible for the cap units to be formed so as to be able to expand or contract while in a state of contact with the upper end portion of the eggs E without moving up or down. More specifically, as is shown in FIG. 11, it is also possible to adopt a structure in which the cap units 12 are formed having, for example, a bellows configuration that enables them to expand or contract so as to track the up-down movement of the eggs E. In addition, an expanding/contracting structure such as, for example, a telescopic structure may also be adopted.

Moreover, in the above-described embodiment, the light shielding unit 7 of each upper light source 51 is used as the cap unit 12, however, it is also possible for a cap unit 12 that maintains the attitude of an egg whose attitude has been adjusted by an attitude adjustment unit 11 to be provided separately from the light shielding unit 7 of each upper light source 51.

Furthermore, in the above-described embodiment, a structure is adopted in which the attitude adjustment units 11 are moved upwards or downwards using the actuator 43 of each lift unit 4, however, it is also possible to adopt a structure in which an actuator that moves the attitude adjustment units 11 upwards or downwards is provided separately from the actuator 43 of each lift unit 4.

In addition to these, in the above-described embodiment, a structure is adopted in which the ascent of the attitude adjustment units 11 is stopped after the attitude of the eggs E has been adjusted by the attitude adjustment units 11, and the lifting units 4 then lift the eggs E above the elastic bodies 11a, however, it is also possible to adopt a structure in which the attitude adjustment units 11 are lowered after the eggs E have been transferred to the lifting units 4.

In the above-described embodiment, a structure is adopted in which the light irradiation units 5 include the upper light sources 51 and the lower light sources 52, and light is irradiated onto the eggs E from both above and below, however, it is also possible to adopt a structure in which the light irradiation units 5 are only provided with either the upper light sources 51 or the lower light sources 52, and light is irradiated onto the eggs E from only one of above or below the eggs E.

In the above-described embodiment, a structure is adopted in which photographic images of the eggs E are captured each time the stage units 4 are rotated, however, it is also possible to adopt a structure in which photographic images of the eggs E are captured without the stage units being rotated.

Moreover, in the above-described embodiment, a structure is adopted in which a plurality of inspection areas, namely, the inspection areas A1 to A3, are set on the conveyance path 2, and the lifting units 4 that lift up a predetermined row of the eggs E are provided in each of the inspection areas A1 to A3, however, it is also possible to provide a plurality of the lifting units 4 in each of the areas A1 to A3 so as to enable the eggs E positioned in mutually different rows of the egg tray 10 to be lifted up in sequence. In addition, by adopting a structure in which a lifting unit 4 is able to be moved to different rows, it is also possible for the eggs E placed in different rows to be lifted up.

Furthermore, in the above-described embodiment, a structure is adopted in which the first through third inspection areas A1 to A3 are divided into two, however, it is also possible for each inspection area to not be divided and to stay as a single inspection area.

In the above-described embodiment, the light emission patterns of the respective inspection areas A1 to A3, as well as the sequence of light emission patterns are the set as the same, however, it is also possible for the light emission patterns to be mutually different from each other, and for the sequence of light emission patterns to be mutually different from each other.

In addition, in the above-described embodiment, a structure is adopted in which the lifting units 4 and the attitude adjustment units 11 lift up the eggs E moving up or down relatively to the egg trays 10, however, it is also possible to adopt a structure in which the eggs E are lifted as a result of the egg trays 10 moving up or down relatively to the lifting units 4 and the attitude adjustment units 11.

Furthermore, in the above-described embodiment, a structure is adopted in which the light irradiation units 5 switch between two light irradiation patterns in which they skip one row of eggs to the next irradiation, however, it is also possible to adopt a structure in which the light irradiation units 5 switch between a plurality of light emission patterns in which two mutually adjacent light sources 51 and 52 do not emit light simultaneously. For example, it is also possible to adopt a structure in which the light irradiation units 5 switch between three light irradiation patterns in which they skip two rows of eggs to the next irradiation. In other words, it is sufficient if a structure is adopted in which the light irradiation units 5 switch between light emission patterns that ensure that eggs E onto which light is not irradiated are interposed between two eggs E onto which light is irradiated. Moreover, it is also possible for all of the light sources 51 and 52 of the light irradiation units 5 to emit light all at once.

Additionally, in addition to a structure in which the egg inspection apparatus 100 lifts a plurality of eggs E located in a predetermined row from the egg tray 10 and then inspects these eggs E, it is also possible, for example, for a plurality of eggs E to be placed in advance in a particular row, and for the plurality of eggs E placed in that row to be inspected in that state. Moreover, it is also possible to adopt a structure in which the egg inspection apparatus 100 causes the conveyance path 2 to operate after a plurality of eggs E have been placed in advance in a particular row, and for the conveyance path 2 to then be stopped in a predetermined inspection area so that the plurality of eggs E can be inspected in that predetermined inspection area.

In addition, various modifications of the embodiments may be made within the scope not deviating from the gist of the present invention.

### [Industrial Applicability]

According to the present invention, it is possible to adjust the attitude of an egg and to then perform an accurate inspection of that egg.

### [Reference Signs List]

- 100: Egg Inspection Apparatus
- 10: Egg Tray
- E: Egg
- 41: Stage Unit
- 43: Actuator
- 4: Lifting Unit
- 5: Light Irradiation Unit
- 6: Imaging Unit
- 11: Attitude Adjustment Unit
- 11a: Plurality of Elastic Bodies
- 12: Cap Unit

## Claims

1. An egg inspection apparatus that inspects an egg held on an egg tray, comprising:
an attitude adjustment unit that adjusts an attitude of the egg by lifting the egg off the egg tray using a plurality of elastic bodies that surround a periphery of the egg;
a cap unit that is placed in contact with an upper end portion of the egg that has been lifted up by the attitude adjustment unit;
a lifting unit that lifts up the egg using the elastic bodies while the cap unit is in contact with the egg;
a light irradiation unit that irradiates light onto the egg that has been lifted by the lifting unit; and
an imaging unit that captures a photographic image of the egg that has been lifted by the lifting unit.

2. The egg inspection apparatus according to Claim 1, wherein the cap unit is formed so as to be able to move up or down while still in contact with the upper end portion of the eggs.

3. The egg inspection apparatus according to either of Claims 1 or 2, wherein the lifting unit comprises:
a stage unit that is in contact with a lower end portion of the eggs; and
an actuator that causes the stage unit to move upwards or downwards, wherein
the attitude adjustment unit is provided so as to be able to move relatively to the stage unit, and is moved up or down by the actuator, and,
when the attitude adjustment unit is moved up by the actuator, the attitude adjustment unit adjusts the attitude of the eggs by lifting up the eggs and, thereafter, the upward movement of the attitude adjustment unit is halted and the stage unit is then moved up by the actuator so that the egg is lifted up above the elastic bodies.
